# EUROPÄISCHE PATENTANMELDUNG

(11) **EP 4 825 420 A2**
(43) Veröffentlichungstag der Anmeldung: **21.10.2026**
(21) Anmeldenummer: 26194506.7
(22) Anmeldetag: 28.09.2023
(51) Int. Cl.: A61M 5/46

(54) **VORRICHTUNG ZUR POSITIONIERUNG EINES INJEKTORS, INJEKTIONSVORRICHTUNG UND VERFAHREN**

(62) Dokumentnummer(n) der früheren Anmeldung(en) nach Art. 76 EPÜ: 23200358.2 / 4 529 939
(71) Anmelder: Heraeus Medical GmbH, 61273 Wehrheim (DE)
(72) Erfinder: Dr. Vogt, Sebastian, 61273 Wehrheim (DE); Dr. Kluge, Thomas, 61273 Wehrheim (DE)
(74) Vertreter: Heraeus IP

(57) **Zusammenfassung**

Die Erfindung betrifft eine Vorrichtung zur Positionierung eines Injektors in der Nähe eines Gelenks sowie eine Vorrichtung zur Injektion eines Stoffes in der Nähe eines Gelenks. Eine Vorrichtung (10) zur Positionierung eines Injektors (50) in der Nähe eines Gelenks (20) umfasst zwei Klemmbacken (11, 12) zur Fixierung einer Hautfalte (22) in einer Klemmposition (15) und eine Schnittstelle (18) zur mechanischen Verbindung eines Injektors (50). Auf diese Weise wird eine sichere, reproduzierbare und einfache Injektion von Stoffen in unmittelbarer Nähe zu einem Gelenk ermöglicht.

## Beschreibung

Die Erfindung betrifft eine Vorrichtung zur Positionierung eines Injektors in der Nähe eines Gelenks, eine Vorrichtung zur Injektion eines Stoffes und ein Verfahren zur Verwendung der Vorrichtung.

Rheumatische Erkrankungen gehören zur den Autoimmunerkrankungen, bei denen das Immunsystem aus noch nicht genau bekannten Ursachen körpereigene Strukturen angreift. Die rheumatoide Arthritis und die Psoriasis-Arthritis gehören zum rheumatischen Formenkreis und sind mit Gelenkentzündungen oder Arthritiden verbunden, die meist chronisch verlaufen und zur fortschreitenden Zerstörung der Gelenke führen können. Für die systemische medikamentöse Therapie der rheumatoiden Arthritis und die Psoriasis-Arthritis stehen eine Reihe von Wirkstoffgruppen zur Verfügung, beispielsweise Analgetika, nicht-steroidale Antiphlogistika, Glucocorticoide und Basistherapeutika (Disease modifying antirheumatic drugs, DMARD) unterteilt. Daneben gibt es moderne Antikörper (Biologicals), welche das α-TNF (α-Tumornekrosefaktor) oder das Interleukin IL-17 blockieren. Mit der systemischen medikamentösen Therapie können jedoch nur relativ geringe Serumkonzentrationen der Wirkstoffe erreicht werden, da anderenfalls unerwünschte Nebenwirkungen auftreten, wie zum Beispiel Schädigungen der Nieren und der Leber. Höhere Serumkonzentrationen werden daher in der Praxis zumindest bei länger andauernden Therapien zumeist vermieden.

Das hat zur Folge, dass am Bestimmungsort, z. B. im Gewebe der Gelenkkapsel, die Wirkstoffkonzentrationen nur sehr niedrig sein können und daher nur ein begrenzter immunmodulierender Effekt möglich ist. Zur Verminderung der chronischen Entzündungsprozesse im Gelenkbereich von Patienten mit rheumatoider Arthritis und mit Psoriasis-Arthritis sowie bei vielen anderen Krankheiten wäre es daher wünschenswert, wenn die entsprechenden Wirkstoffe lokal in der Nähe der Gelenkkapsel und/oder betroffener Sehnen bzw. Sehnenansätze und insbesondere direkt am betroffenen Körperstruktur appliziert werden könnten, um eine lokal hohe Konzentration dieser Wirkstoffe zu erreichen, aber gleichzeitig eine hohe systemische Wirkstoffkonzentration zu vermeiden.

Auch die Gabe schmerzstillender Medikamente, beispielsweise im Falle einer aktivierten Arthrose, in die direkte Nähe des Zielortes, beispielsweise eines Gelenks, wäre wünschenswert. Daneben ist eine Vielzahl weiterer Krankheitsbilder bekannt, bei denen mit systemischer Gabe von Wirkstoffen lediglich vergleichsweise geringe Konzentrationen am jeweiligen Zielort, erreichbar sind, beispielsweise an oder in Gelenken, obwohl höhere lokale Konzentrationen wünschenswert wären.

Das Gewebe der Gelenkkapsel ist aufgrund der dort befindlichen Nozizeptoren sehr schmerzempfindlich. Eine Verletzung der Gelenkkapsel sollte daher vermieden werden. Weiterhin sollte eine Perforation der Gelenkkapsel verhindert werden, um eine Infektion der Gelenkkapsel und/oder der Synovialflüssigkeit zu vermeiden.

Eine einfache periartikuläre Injektion mit einer Spritze oder mit beispielsweise einem aus der Diabetes-Behandlung bekannten Injektor-Pen ist grundsätzlich möglich, aber nicht praktikabel, da es sehr schwierig ist, nur das auf der Gelenkkapsel aufliegende Gewebe mit der Injektionskanüle zu perforieren und dort eine geringe Menge eines Wirkstoffes zu injizieren, ohne dass die Gelenkkapsel berührt oder verletzt würde. Auch ein Einstechen mit einer grundsätzlich nur sehr geringen Tiefe mit dem Hintergrund, einen sicheren Abstand zwischen der Kanüle und der Gelenkkapsel zu gewährleisten, kann dieses Problem nicht lösen, da das die Gelenkkapseln überdeckende Gewebe hinsichtlich seiner Dicke von Gelenk zu Gelenk und von Patient zu Patienten sehr unterschiedlich ist.

Der Erfindung liegt die Aufgabe zugrunde, eine verbesserte Möglichkeit zu schaffen, einen Stoff in die Nähe eines Gelenks zu injizieren. Insbesondere soll eine Verletzung der Gelenkkapsel dabei verhindert werden.

Die Aufgabe wird gelöst durch die Vorrichtung zur Positionierung eines Injektors in der Nähe eines Gelenks gemäß Anspruch 1 und durch die Vorrichtung zur Injektion eines Stoffes in der Nähe eines Gelenks gemäß dem nebengeordneten Anspruch. Vorteilhafte Ausgestaltungen sind in den Unteransprüchen angegeben.

Zur Lösung der Aufgabe dient eine Vorrichtung zur Positionierung eines Injektors in der Nähe eines Gelenks. Die Vorrichtung umfasst zwei Klemmbacken zur Fixierung einer Hautfalte, insbesondere in einer Klemmposition, und eine Schnittstelle zur mechanischen Verbindung eines Injektors.

Die Erfindung basiert auf der Erkenntnis, dass eine Hautfalte in der Nähe eines Gelenks, beispielsweise neben einem Gelenk, eine reproduzierbare Injektion eines Stoffes in der Nähe des Gelenks ermöglicht, wobei das Risiko einer Verletzung der Gelenkkapsel praktisch ausgeschlossen werden kann. Unabhängig von der konkreten Anatomie des Patienten ist es durch Fixieren einer Hautfalte möglich, eine sichere Position für das Setzen einer Kanüle oder einer anderen Injektionseinrichtung zu gewährleisten. Die Vorrichtung erlaubt die Positionierung des Injektors in einer definierten Position in der Nähe eines Gelenks, so dass auf definierte Weise ein Stoff in der Nähe des Gelenks injiziert werden kann. Schmerzen und Infektionen werden verhindert, zudem wird die Akzeptanz der Vorrichtung bzw. der Injektion erhöht.

Selbstverständlich kann die Vorrichtung auch die Positionierung an anderen Positionen eines menschlichen oder tierischen Körpers erlauben.

Die zwei Klemmbacken sind dazu eingerichtet, eine Hautfalte in der Nähe eines Gelenks zu fixieren. Die Klemmbacken können eine geeignete Hautfalte insbesondere ergreifen bzw. ziehen und fixieren. Die Hautfalte und ggf. unter der Haut befindliches Binde- und/oder Fettgewebe kann zwischen den Klemmbacken eingeklemmt und auf diese Weise fixiert werden. Die Klemmbacken wirken somit wie eine Klammer. Das Gelenk ist insbesondere ein Gelenk eines menschlichen oder tierischen Körpers.

Ein Injektor ist eine Vorrichtung zur insbesondere subkutanen Injektion eines Stoffes, insbesondere eines Wirkstoffes. Ein Injektor weist eine Ausgabeeinrichtung auf, mit der der Stoff ausgegeben werden kann. Die Ausgabeeinrichtung kann beispielsweise als Kanüle ausgebildet sein. Alternativ kann der Injektor, wie im Falle eines Jet-Injektors bzw. nadelfreien Injektors, ohne Kanüle arbeiten. In diesem Fall kann die Ausgabeeinrichtung eine Ausgabeöffnung umfassen, durch die ein Strahl des Stoffes mit hohem Druck ausgegeben werden kann. Ein Injektor kann beispielsweise als Pen vorliegen, z. B. nach Art eines Insulin-Pens. Grundsätzlich kann der Injektor auch für eine intradermale oder interkutane Injektion eingerichtet sein.

Die Vorrichtung dient dazu, den Injektor, relativ zum Körper zu positionieren. Auf diese Weise kann die Ausgabeeinrichtung des Injektors mit der Ausgabeöffnung, über welche der zu injizierende Stoff aus dem Injektor ausgegeben werden kann, in einer definierten Position in Bezug zum Körper, beispielsweise zum Gelenk, fixiert werden. Typischerweise soll der Stoff in der Nähe des Gelenks injiziert werden. Die Positionierung erfolgt insbesondere derart, dass die Injektion in einen definierten Bereich der Hautfalte erfolgen kann. Auf diese Weise kann eine Verletzung der Gelenkkapsel praktisch ausgeschlossen werden. Zudem wird eine einfache, wiederholbare und auch von medizinischen Laien wie dem Patienten selbst durchführbare Injektion ermöglicht. Diese kann auch bei Bedarf ausgeführt werden, also beispielsweise kann bei akuten Schmerzen ein Analgetikum selbst gegeben werden.

Die Schnittstelle dient dazu, den Injektor mechanisch mit der Vorrichtung zu verbinden. Die Schnittstelle ist insbesondere dazu eingerichtet, ein Formelement des Injektors in einer definierten Position zu halten. Auf diese Weise kann die relative Position des Injektors zur Vorrichtung und damit die relative Position der Ausgabeeinrichtung zur Hautfalte auf definierte Weise eingestellt werden. Beispielsweise hat die Schnittstelle einen Einsteckbereich, in den ein Gehäuseabschnitt des Injektors eingesteckt werden kann und so beispielsweise kraftschlüssig gehalten werden kann.

Die Klemmbacken sind relativ zueinander beweglich. Dabei kann eine Klemmbacke fest sein und die andere Klemmbacke kann beweglich sein oder es können beide Klemmbacken beweglich sein. Auf diese Weise kann ein Abstand zwischen den Klemmbacken vergrößert werden, um die Hautfalte zwischen den Klemmbacken anzuordnen bzw. die Klemmbacken über die Hautfalte zu schieben oder um die Hautfalte aus der Fixierung zu lösen. Auf diese Weise kann zudem ein Abstand zwischen den Klemmbacken verringert werden, um die Hautfalte zu fixieren. Die Klemmposition ist die Position, in der sich die Hautfalte im fixierten Zustand befindet. Die Klemmposition befindet sich typischerweise zwischen den Klemmbacken.

Typischerweise weist jede Klemmbacke eine Klemmfläche auf. Die Klemmfläche ist die Oberfläche der Klemmbacke, die im fixierten Zustand der Hautfalte die Haut kontaktiert. Die Klemmfläche kann strukturiert ausgebildet sein, um ein Rutschen der Haut senkrecht zur Klemmfläche zu verhindern. Die Klemmflächen der Klemmbacken können im geschlossenen Zustand der Klemmbacken ungefähr parallel zueinander ausgerichtet sein.

Insbesondere ist eine erste Klemmbacke der zwei Klemmbacken mechanisch mit der Schnittstelle verbunden. Insbesondere ist eine zweite Klemmbacke der zwei Klemmbacken schwenkbar mit der ersten Klemmbacke verbunden.

In einer Ausführungsform ist die Vorrichtung fest mit dem Injektor verbunden. Die Schnittstelle stellt somit eine dauerhafte, nicht zerstörungsfrei lösbare Verbindung zwischen Vorrichtung und Injektor dar. In einer Ausführungsform ist die Vorrichtung einstückig mit dem Injektor hergestellt. Die Schnittstelle wird dann entsprechend durch die Verbindung zwischen dem als Injektor dienenden Bereich und dem als Vorrichtung zur Positionierung des Injektors dienenden Bereich mit den Klemmbacken bereitgestellt.

In einer Ausgestaltung ist die Schnittstelle zur Herstellung einer lösbaren Verbindung des Injektors mit der Vorrichtung eingerichtet. Der Injektor kann also reversibel mit der Vorrichtung verbunden werden. Somit ist ein zerstörungsfreies, insbesondere manuelles Lösen der Verbindung möglich. Die Vorrichtung kann beispielsweise mit jeweils unterschiedlichen Injektoren mehrfach verwendet werden. Z. B. ist die Schnittstelle zur Herstellung einer Steck- und/oder Schraubverbindung eingerichtet.

Es ist möglich, die Vorrichtung zusammen mit handelsüblichen Injektoren zu verwenden. Durch die Schnittstelle kann rasch und einfach eine lösbare mechanische Verbindung hergestellt werden. Beispielsweise sind Injektoren bekannt, die eine wechselbare Kappe mit einer Kanüle aufweisen. In diesem Fall kann die Schnittstelle beispielsweise zur Befestigung dieser Kappe eingerichtet sein, etwa durch Aufstecken auf diese Kappe.

In einer Ausgestaltung weist die Schnittstelle eine Anschlagfläche auf, an der ein Formelement des Injektors in einem definierten Abstand zu der Klemmfläche anliegen kann.

Im Sinne der Erfindung werden auch punkt- oder linienförmige Anschläge als Anschlagfläche bezeichnet werden, da in der Realität i. d. R. immer ein flächiger Kontakt vorliegt. Größere Flächen sind jedoch zu bevorzugen. Die Anschlagfläche kann im Wesentlichen quer zur Injektionsrichtung ausgerichtet sein. Die Injektionsrichtung meint die Richtung, entlang welcher der Stoff bei der Injektion bewegt wird, also beispielsweise die Ausrichtung einer Ausgabeeinrichtung wie z. B. einer Kanüle. Abweichungen von wenigen Grad können akzeptabel sein. Der Anschlag kann im Wesentlichen parallel zu einer Klemmfläche einer der Klemmbacken ausgerichtet sein. Auch hier können Abweichungen von wenigen Grad akzeptabel sein.

Das Formelement des Injektors kann beispielsweise eine zur Ausgabeeinrichtung hin weisende vordere und/oder stirnseitige Fläche sein, beispielsweise eine um die Achse der Ausgabeeinrichtung umlaufende kreisringförmige Vorderfläche des Injektors.

Diese Ausgestaltung erlaubt es, den Injektor beispielsweise vorwärts, entlang der Richtung der Ausgabeeinrichtung, in Richtung der Vorrichtung zu bewegen, bis der Injektor die Anschlagfläche kontaktiert. In dieser Position hat dann die Ausgabeeinrichtung, z. B. die Spitze der Kanüle, einen definierten Abstand zu zumindest einer Klemmbacke. So kann die Eindringtiefe der Kanüle bzw. die Position des Jets in Bezug zur Haut auf einfache Weise genau und reproduzierbar eingestellt werden. Eine besonders sichere und unkomplizierte Bedienung ist somit auch durch den Patienten selbst möglich.

In einer Ausgestaltung weist die Vorrichtung zwischen der Schnittstelle und einer ersten Klemmbacke der zwei Klemmbacken einen durchgehenden Kanal auf. Der Kanal verläuft insbesondere etwa senkrecht zur Klemmfläche der ersten Klemmbacke. Der Kanal mündet typischerweise in einer Durchgangsöffnung in der ersten Klemmbacke. Die Durchgangsöffnung verläuft durch die Klemmfläche der ersten Klemmbacke hindurch, sodass die Ausgabeeinrichtung durch die Klemmfläche hindurch bewegt und/oder dort angeordnet werden kann. Die Schnittstelle verläuft typischerweise in Umfangsrichtung um den Kanal herum.

Der Kanal dient dazu, die Ausgabeeinrichtung aufzunehmen. Es kann beispielsweise eine Verbindung zwischen dem Injektor und der Vorrichtung hergestellt werden, bei der zumindest ein Teil der Ausgabeeinrichtung durch den Kanal bewegt, z. B. geschoben, wird. Im Falle einer Kanüle kann eine Kanülenspitze beispielsweise bis über die Klemmfläche hinaus bis in den Raum zwischen den Klemmbacken bewegt werden. Im Falle eines Jet-Injektors kann eine Ausgabeöffnung beispielsweise vor der Klemmfläche der ersten Klemmbacke oder auf Höhe der Klemmfläche verbleiben.

In einer Ausführungsform ist der Kanal in einem Winkel von mindestens 0° und/oder höchstens 50° zu einer Ebene ausgerichtet, die senkrecht zu einer Klemmfläche der ersten Klemmbacke verläuft. Die Ausrichtung des Kanals wird in Bezug zu seiner Mittelachse gemessen. Diese Ausrichtung erlaubt es aufgrund der zugrundeliegenden Geometrie, mit besonders großer Sicherheit eine Verletzung der Gelenkkapsel auszuschließen.

Die Länge des Kanals ist insbesondere so gewählt, dass bei Verwendung eines handelsüblichen Injektors eine gewünschte Eindringtiefe der Ausgabeeinrichtung in die Hautfalte erfolgt. Beispielsweise kann es gewünscht sein, dass die Spitze einer Kanüle sich etwa mittig zwischen den beiden Klemmbacken und damit mittig in der Hautfalte befindet.

In einer Ausführungsform weist die Vorrichtung auch zwischen der Schnittstelle und der zweiten Klemmbacke einen durchgehenden Kanal auf. Auf diese Weise kann die Vorrichtung gedreht werden und in beiden Richtungen verwendet werden.

In einer Ausführungsform hat die erste Klemmbacke eine Durchgangsöffnung.

In einer Ausgestaltung verjüngt sich der Kanal von der Schnittstelle hin zur ersten Klemmbacke. Es ist so ein größerer Flächenbereich vorhanden, in den die Ausgabeeinrichtung eingeführt werden kann. Der Kanal kann auch so eingerichtet sein, dass die Ausgabeeinrichtung durch eine Wandung des Kanals zur Durchgangsöffnung hin geführt werden kann. Auf diese Weise wird ein Einführen der Ausgabeeinrichtung des Injektors in den Kanal und/oder ein Bewegen der Ausgabeeinrichtung durch den Kanal erleichtert. Eine Fehlbedienung wird verhindert.

Insbesondere hat die Durchgangsöffnung einen Durchmesser, der wenigstens einem Vielfachen, beispielsweise dem Doppelten, dem Dreifachen oder dem Fünffachen des Durchmessers der Ausgabeeinrichtung entspricht. Im Falle eines sich verjüngenden Kanals entspricht diese Durchgangsöffnung typischerweise der minimalen Weite des Kanals. Auf diese Weise kann eine Fehlbedienung verbessert verhindert werden.

In einer Ausgestaltung beträgt ein Abstand zwischen einer Außenkante der ersten Klemmbacke oder einer Außenkante einer zweiten Klemmbacke der zwei Klemmbacken und einem Mittelpunkt einer mit dem Kanal verbundenen Durchgangsöffnung in der ersten Klemmbacke mindestens 1 mm und/oder höchstens 4 mm. Der Abstand wird typischerweise entlang der Richtung der Klemmfläche der ersten Klemmbacke gemessen. Der Abstand kann mindestens 1,5 mm, insbesondere mindestens 2 mm und/oder höchstens 3,5 mm, insbesondere höchstens 3 mm betragen.

Es hat sich gezeigt, dass auf diese Weise bei nahezu allen Patienten eine geeignete Hautfalte für eine Injektion fixiert werden kann und damit eine verlässliche und reproduzierbare Injektion möglich ist.

In einer Ausgestaltung weist die Vorrichtung eine erste Einstelleinrichtung auf, mit der die relative Position des Injektors in Bezug zu zumindest einer Klemmbacke eingestellt werden kann.

Beispielsweise ist die Schnittstelle so am Rest der Vorrichtung befestigt, dass unterschiedliche Positionen der Schnittstelle in Bezug zu einer oder beiden Klemmbacken eingestellt werden können. Auf diese Weise kann eine Anpassung erfolgen, um beispielsweise eine Kanüle mit einer bestimmten Länge zu verwenden. Im Falle einer längeren Kanüle kann der Abstand des Injektors zur Klemmposition beispielsweise erhöht werden, damit eine gleichbleibende Einstechtiefe gewährleistet wird. Auch kann eine individuelle Anpassung an die Dicke der Hautfalte erfolgen, die sich von Stelle zu Stelle und von Patient zu Patient ggf. deutlich unterscheiden kann.

Um die Vorrichtung an der Hautfalte zu fixieren, ragt die Kanüle insbesondere nicht über die Klemmfläche der ersten Klemmbacke hinaus. So besteht kein Risiko einer Verletzung. Wenn der Injektor mechanisch mit der Vorrichtung verbunden ist, ragt die Kanüle bevorzugt um wenigstens 1 mm und/oder höchstens 3 mm oder 4 mm, beispielsweise um ungefähr 2 mm, über die Klemmfläche der ersten Klemmbacke hinaus. Die Hautfalte ist bevorzugt ungefähr doppelt so dick wie dieser Zahlenwert, sodass in die Mitte der Hautfalte injiziert wird.

In einer Ausgestaltung umfasst die erste Einstelleinrichtung ein Gewinde. Die relative Position wird insbesondere bestimmt durch die Drehposition zweier miteinander korrespondierender Gewindeteile. Je nachdem, wie weit ein Gewindeteil in Bezug zum anderen Gewindeteil gedreht wird, ragt beispielsweise die Spitze der Ausgabeeinrichtung weiter oder weniger weit nach vorn, z. B. über die erste Klemmbacke hinaus. Insbesondere verläuft die Drehachse des Gewindes entlang der Richtung der Ausgabeeinrichtung und/oder quer zur Klemmbacke.

In einer Ausführungsform umfasst die Einstelleinrichtung ein fest mit der ersten Klemmbacke verbundenes erstes Gewindeelement mit einem Innen- oder Außengewinde sowie ein mit dem ersten Gewindeelement korrespondierendes zweites Gewindeelement mit einem entsprechend anderen Gewinde. Das zweite Gewindeelement kann mit der Schnittstelle verbunden sein, beispielsweise einstückig, sodass die Schnittstelle durch relatives Drehen der beiden Gewindeteile in Bezug zur ersten Klemmbacke bewegt werden kann. Ein Gewindeelement ist ein Bauteil mit einem Gewinde.

In einer Ausgestaltung weist die Vorrichtung eine zweite Einstelleinrichtung auf, mit welcher ein minimaler Abstand zwischen den Klemmbacken, insbesondere in der Klemmposition, eingestellt werden kann.

Die zweite Einstelleinrichtung kann beispielsweise als Stellschraube, z. B. in Form einer Madenschraube, ausgebildet sein, die in einem Gewinde in einer der Klemmbacken befestigt ist und die andere Klemmbacke kontaktiert. Je nach Position der Stellschraube kann so ein minimaler Abstand zwischen den Klemmbacken eingestellt werden.

Es kann so verhindert werden, dass die Klemmbacken eine Hautfalte zu stark zusammendrücken oder quetschen. Ein schmerzfreies Fixieren der Hautfalte, zumindest für die nötige Zeitdauer der Injektion, etwa im Bereich einiger Sekunden, kann so gewährleistet werden. Zudem kann eine Anpassung an beispielsweise eine Anatomie und/oder einen Zustand des Bindegewebes des Patienten erfolgen.

In einer Ausgestaltung umfasst die Vorrichtung ein Betätigungselement, welches manuell betätigt werden kann, um einen Abstand zwischen den Klemmbacken in der Klemmposition zu verringern und/oder zu vergrößern. Das Betätigungselement ist insbesondere dazu eingerichtet die Klemmbacken von einer geschlossenen Position in eine geöffnete Position zu bewegen, um die Klemmposition freizugeben und die Hautfalte darin anordnen zu können. Dies ermöglicht eine besonders einfache Fixierung der Hautfalte. Das Betätigungselement kann auch geeignet sein, die Klemmbacken wieder in die geschlossene Position zu bewegen. Mit "die Klemmbacken bewegen" ist gemeint, dass eine Klemmbacke oder beide Klemmbacken so bewegt werden, dass eine Relativbewegung zwischen den Klemmbacken erfolgt.

In einer Ausgestaltung ist das Betätigungselement als Hebel ausgestaltet, wobei ein Drücken des Hebels bewirkt, dass der Abstand vergrößert wird. Insbesondere ist der Hebel mechanisch mit einer Klemmbacke, insbesondere der zweiten Klemmbacke, verbunden. Bevorzugt ist der Hebel einstückig mit einer Klemmbacke und/oder als Verlängerung der Klemmbacke hergestellt. So kann die Klemmbacke durch Betätigen des Hebels auf einfache Weise bewegt werden.

In einer Ausführungsform ist ein zweiter Hebel an der jeweils anderen Klemmbacke angeordnet. Auf diese Weise können die Hebel nach Art einer Wäscheklammer zusammengedrückt werden, um den Abstand zwischen den Klemmbacken in der Klemmposition zu verringern. Dies ermöglicht eine besonders einfache Bedienung, etwa mit Daumen und Zeigefinger.

In einer Ausführungsform sind die Klemmbacken relativ zueinander um eine Drehachse schwenkbar. Die Drehachse kann eine physische Achse, beispielsweise eine Achse aus Kunststoff oder Metall, oder eine nicht physisch vorhandene gedachte (virtuelle) Achse sein. Im letzteren Fall sind die Klemmbacken auch ohne physische Achse derart miteinander verbunden, dass ein relatives Schwenken erfolgen kann.

In einer Ausgestaltung ist der Hebel in einem Winkel von wenigstens 60° und/oder höchstens 120° zu zumindest einer der Klemmbacken ausgerichtet. Dies ermöglicht, dass der Hebel etwa parallel oder nur in einem kleinen Winkel zu der Haupterstreckungsrichtung des Injektors ausgerichtet ist. Auf diese Weise ist es möglich, mit einer Hand den Injektor, ggf. mitsamt der daran befestigten Vorrichtung, zu halten und gleichzeitig den Hebel zu betätigen, um die Hautfalte zu fixieren bzw. die Fixierung der Hautfalte vorzubereiten. Zudem kann der Körper des Injektors als zweiter Hebel dienen, gegenüber dem der Hebel zu bewegen ist. So ist es nicht notwendig, einen separaten zweiten Hebel vorzusehen.

Der Hebel muss nicht gerade sein. Im Falle eines gekrümmten Hebels kann beispielsweise eine mittlere Ausrichtung des Hebels oder eine Verbindung eines Anfangspunktes mit einem Endpunkt des Hebels zur Messung des Winkels herangezogen werden. Beispielsweise kann der Hebel etwa senkrecht zur Klemmbacke ausgerichtet sein oder der Winkel kann wenigstens 70° oder wenigstens 80° und/oder höchstens 110° oder höchstens 100° betragen. Bevorzugt beträgt der Winkel 85° oder größer. So wird die Bedienung weiter vereinfacht.

In einer Ausführungsform ist der Hebel im Wesentlichen parallel zu einer der Klemmbacken ausgerichtet, insbesondere zu der Klemmbacke, mit der der Hebel mechanisch verbunden ist. Insbesondere ist der Hebel mit einer Klemmbacke mechanisch verbunden oder einstückig hergestellt und/oder im Wesentlichen senkrecht zu dieser Klemmbacke ausgerichtet.

In einer Ausgestaltung weist die Vorrichtung ein Federelement auf, welches die Klemmbacken in eine geschlossene Position vorspannt. Die geschlossene Position meint die Position, in der die Klemmbacken in der Klemmposition den kleinstmöglichen Abstand zueinander aufweisen. Dieser kleinstmögliche Abstand ist von der Konstruktion der Klemmbacken abhängig. Es ist möglich, aber nicht notwendig, dass sich die Klemmbacken in dieser relativen Position unmittelbar kontaktieren. Das Federelement ist bevorzugt ein elastisch verformbares Element.

Diese Ausgestaltung hat den Effekt, dass eine Kraft ausgeübt wird, um die Klemmbacken in der Klemmposition zusammenzudrücken. Befindet sich eine Hautfalte in der Klemmposition, werden die Klemmbacken durch das Federelement an der Hautfalte fixiert bzw. die Hautfalte wird zwischen den Klemmbacken fixiert. Auf diese Weise kann die Vorrichtung ohne Ausüben einer manuellen Kraft am Körper fixiert werden. Es wird dafür keine Hand benötigt.

In einer Ausgestaltung ist die Vorrichtung einstückig hergestellt. In einer Ausgestaltung ist die Vorrichtung als Kunststoffteil hergestellt. Beispielsweise ist die Vorrichtung als Spritzgussteil hergestellt. Auf diese Weise kann die Vorrichtung besonders einfach und kostengünstig hergestellt werden. Es hat sich herausgestellt, dass alle Funktionselemente der Vorrichtung in einer einstückigen Vorrichtung auf einfache Weise bereitgestellt werden können.

Ein weiterer Aspekt der Erfindung ist eine Vorrichtung zur Injektion eines Stoffes in der Nähe eines Gelenks. Diese umfasst die erfindungsgemäße Vorrichtung zur Positionierung eines Injektors und einen Injektor zur Injektion eines Stoffes. Der Injektor ist mit der Schnittstelle der Vorrichtung zur Positionierung des Injektors verbunden oder verbindbar. Alle Merkmale, Vorteile und Wirkungen der eingangs genannten Vorrichtung gelten auch für die Vorrichtung gemäß diesem Aspekt und umgekehrt.

Der Injektor kann fest, also nicht zerstörungsfrei lösbar, mit der Schnittstelle verbunden sein. Hierbei wird eine direkt einsetzbare Vorrichtung zur Verfügung gestellt. Alternativ kann der Injektor manuell lösbar mit der Schnittstelle verbunden sein. Die Vorrichtung zur Positionierung kann somit mehrfach, beispielsweise mit unterschiedlichen Injektoren, verwendet werden. Die Vorrichtung zur Injektion kann auch als System mit einer Vorrichtung zur Positionierung und einem Injektor bezeichnet werden.

Der Injektor kann eine Einstelleinrichtung zum Einstellen einer zu injizierenden Menge des Stoffes, beispielsweise eines Volumens, eingerichtet sein. Typischerweise ist der Injektor zur Injektion eines Volumens im Bereich einiger µL, beispielsweise zwischen 5 µL und 50 µL, eingerichtet.

Die Vorrichtung bzw. das System kann ferner einen zu injizierenden Stoff enthalten, beispielsweise einen pharmazeutischen Wirkstoff. Der Stoff kann in einer Verpackung verpackt sein.

Ein weiterer Aspekt der Erfindung ist ein Verfahren zur Verwendung der erfindungsgemäßen Vorrichtung zur Positionierung eines Injektors. Das Verfahren umfasst das Fixieren einer Hautfalte mit den zwei Klemmbacken der Vorrichtung. Alle Merkmale, Vorteile und Wirkungen der eingangs genannten Vorrichtung zur Positionierung eines Injektors gelten auch für das Verfahren und umgekehrt.

Insbesondere wird eine Hautfalte in der Nähe eines Gelenks fixiert. Die Hautfalte wird beispielsweise von einem Hautareal gebildet, das eine Gelenkkapsel überdeckt oder an eine Gelenkkapsel angrenzt. In einer Ausführungsform ist das Gelenk ein Gelenk eines Tieres. In einer Ausführungsform ist das Gelenk ein Gelenk eines Menschen.

Das Verfahren kann zur Vorbereitung einer Injektion dienen. Insbesondere umfasst das Verfahren ferner die Injektion eines Stoffes in die Hautfalte mit einem mit der Schnittstelle der Vorrichtung mechanisch verbundenen Injektor. In diesem Fall kann das Verfahren auch als Verfahren zur Injektion eines Stoffes bezeichnet werden. Insbesondere befindet sich die Hautfalte und/oder erfolgt die Injektion in der Nähe eines Gelenks. Insbesondere wird der Stoff subkutan injiziert.

Beispielsweise enthält der Stoff einen Entzündungshemmer, ein Antirheumatikum, ein Biological und/oder einen schmerzstillenden Wirkstoff. Beispielsweise wird das Verfahren bei rheumatoider Arthritis, Psoriasis Arthritis und bei Arthrose verwendet.

Das Verfahren kann ferner einen oder mehrere der folgenden Schritte umfassen:
- Beeinflussung einer relativen Position des Injektors in Bezug zu zumindest einer Klemmbacke mittels der ersten Einstelleinrichtung,
- Einstellen eines minimalen Abstands zwischen den Klemmbacken in der Klemmposition mittels der zweiten Einstelleinrichtung,
- Ergreifen einer Hautoberfläche mittels der zwei Klemmbacken unter Bildung einer Hautfalte,
- Aufstecken einer Kappe auf einen Injektorkörper zur Bereitstellung des Injektors,
- Bewegen einer Ausgabeeinrichtung des Injektors durch den Kanal,
- Positionierung einer Ausgabeeinrichtung in Bezug zur Hautfalte derart, dass eine Injektion in die Hautfalte möglich ist, z. B. durch Einführen einer Kanüle in die Hautfalte,
- Entfernung der Klemmbacken unter Freisetzung der geklemmten Haut,
- Entfernung der Ausgabeeinrichtung, z. B. mit dem Injektor.

Nachfolgend werden Ausführungsbeispiele der Erfindung auch anhand von Figuren näher erläutert. Merkmale der Ausführungsbeispiele können einzeln oder in einer Mehrzahl mit den beanspruchten Gegenständen kombiniert werden, sofern nichts Gegenteiliges angegeben ist. Die beanspruchten Schutzbereiche sind nicht auf die Ausführungsbeispiele beschränkt.

Es zeigen:

| | |
|---|---|
| Figur 1: | eine Vorrichtung zur Injektion eines Stoffes im Einsatz, |
| Figur 2: | eine Vorrichtung zur Injektion eines Stoffes, |
| Figur 3: | eine Schnittzeichnung einer Vorrichtung zur Positionierung eines Injektors, |
| Figur 4: | eine Vorrichtung zur Positionierung eines Injektors in einer geöffneten Position, |
| Figur 5: | eine Vorrichtung zur Positionierung eines Injektors im Einsatz, |
| Figur 6: | eine Schnittzeichnung einer Vorrichtung zur Positionierung eines Injektors im Einsatz, |
| Figur 7: | eine weitere Ausführungsform einer Vorrichtung zur Positionierung eines Injektors, |
| Figur 8: | eine weitere Ausführungsform einer Vorrichtung zur Positionierung eines Injektors, |
| Figur 9: | eine weitere Ausführungsform einer Vorrichtung zur Positionierung eines Injektors, |
| Figur 10: | eine weitere Ausführungsform einer Vorrichtung zur Positionierung eines Injektors, sowie |
| Figur 11: | eine Abfolge von Schritten bei der Verwendung einer Vorrichtung zur Positionierung eines Injektors. |

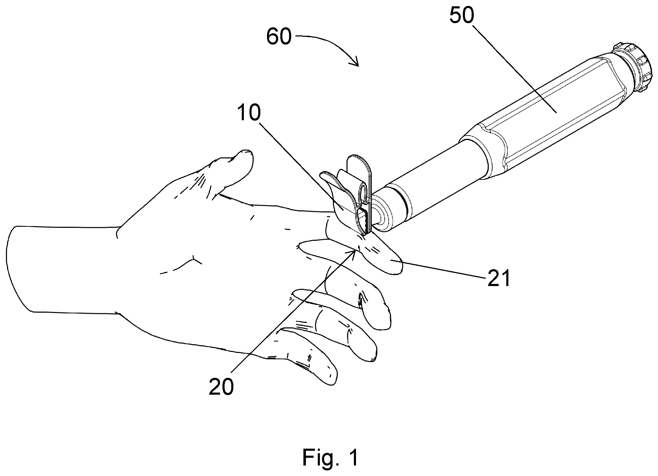

Figur 1 zeigt eine Vorrichtung 60 zur Injektion eines Stoffes in der Nähe eines Gelenks 20. Diese umfasst eine Vorrichtung 10 zur Positionierung eines Injektors 50 in der Nähe eines Gelenks 20 sowie einen mit dieser Vorrichtung 10 mechanisch verbundenen Injektor 50. Die Vorrichtung 10 zur Positionierung eines Injektors 50 positioniert den Injektor 50 in der Nähe eines Gelenks 20, nämlich beispielhaft eines Fingergelenks eines Zeigefingers 21 eines Patienten. Der Injektor 50 kann ein handelsüblicher Injektor 50 sein, beispielsweise nach Art eines Insulin-Pens. Beispielsweise dient der Injektor 50 zur Dosierung eines Medikaments für die Rheuma-Basistherapie oder eines Analgetikums, um im Gelenk 20 eine hohe Wirkstoffkonzentration zu erreichen, gleichzeitig die systemische Konzentration aber niedrig zu halten, um unerwünschte Nebenwirkungen zu minimieren.

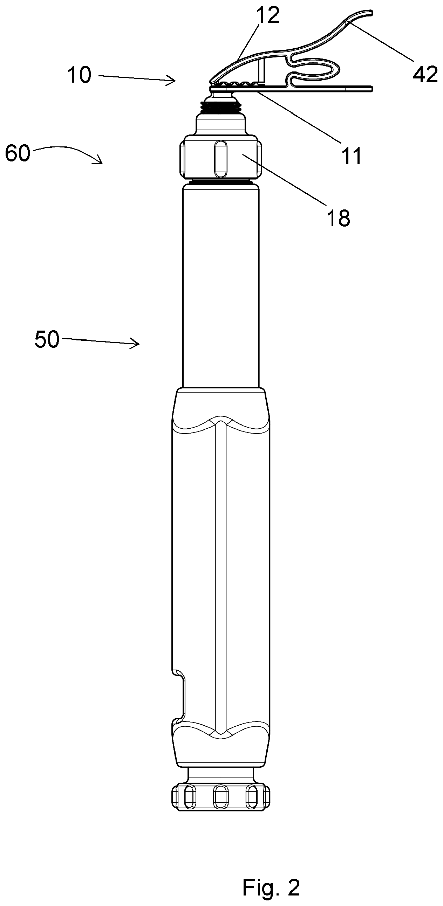

Figur 2 zeigt die Vorrichtung 60 zur Injektion eines Stoffes in einer Seitenansicht. Diese ist zusammengesetzt aus dem Injektor 50 und einer Vorrichtung 10 zur Positionierung des Injektors 50, die zwei Klemmbacken 11, 12, eine Schnittstelle 18 zur mechanischen Befestigung des Injektors 50 sowie einen Hebel 42 umfasst, der mit einer der Klemmbacken 11, 12 verbunden ist.

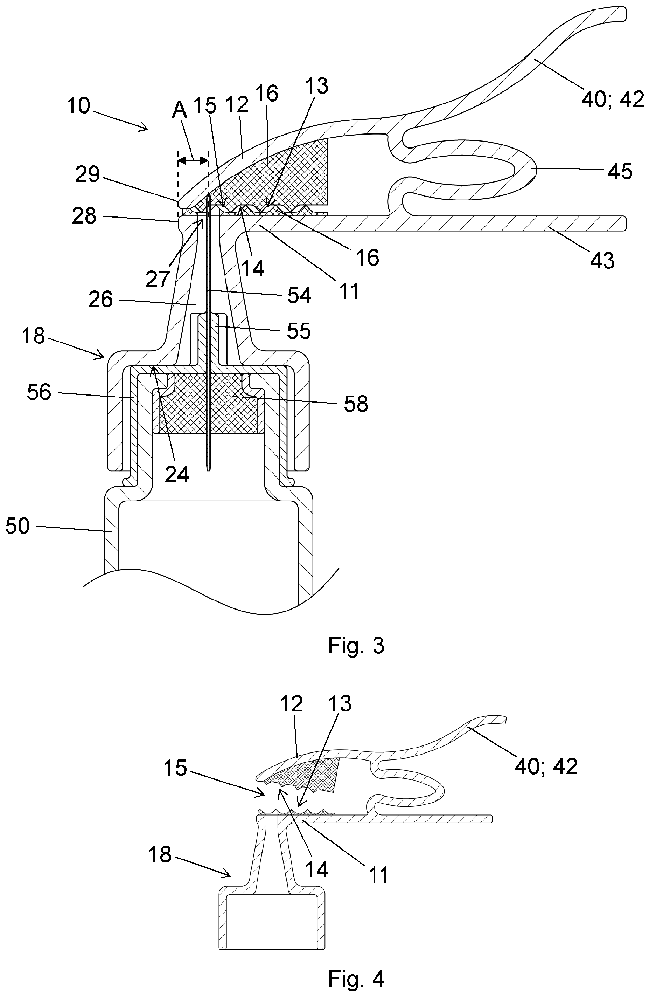

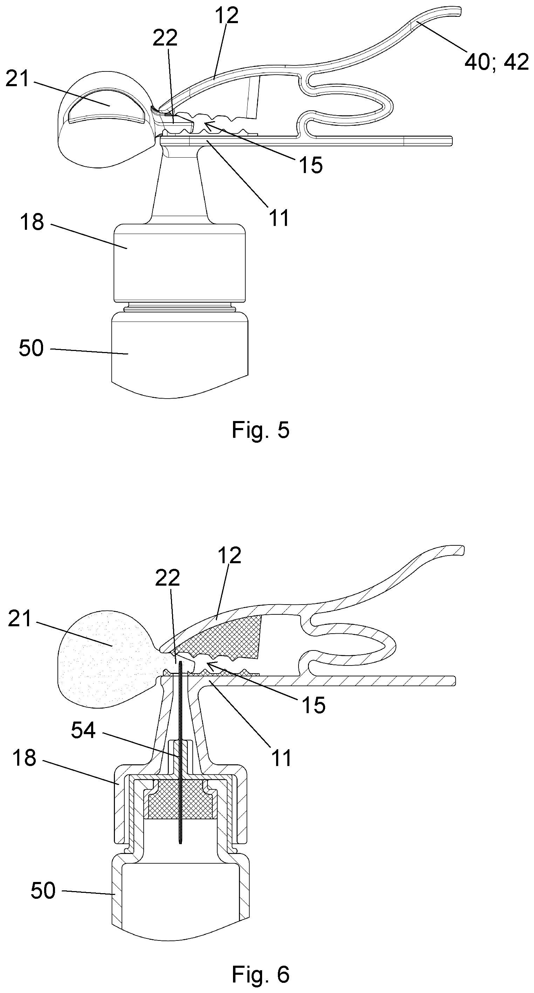

Figur 3 zeigt eine Vorrichtung 10 zur Positionierung des Injektors 50 in einer vergrößerten Schnittdarstellung. Eine beispielhaft untere erste Klemmbacke 11 und eine beispielhaft obere zweite Klemmbacke 12 wirken zusammen, um eine Hautfalte eines Patienten zu fixieren. Zwischen den Klemmbacken 11, 12 befindet sich dementsprechend die Klemmposition 15, in der die Hautfalte eingeklemmt werden kann (siehe Fig. 5 und 6).

Die zweite Klemmbacke 12 ist schwenkbar in Bezug zur ersten Klemmbacke 11 gelagert. Durch Betätigung des als Hebel 42 ausgestalteten Betätigungselements 40, das hier beispielhaft einstückig mit der zweiten Klemmbacke 12 ausgebildet ist, kann die zweite Klemmbacke 12 im links dargestellten Bereich nach oben bewegt werden und gleichzeitig im rechts dargestellten Bereich nach unten bewegt werden. Auf diese Weise kann eine geöffnete Position der Klemmbacken 11, 12 erreicht werden, in der die Klemmposition 15 zugänglich ist. Dies ist in Figur 4 dargestellt. Das Betätigungselement 40 erleichtert somit das Öffnen der Klemmbacken 11, 12. Die Klemmposition 15 befindet sich zwischen der Klemmfläche 13 der ersten Klemmbacke 11 und der Klemmfläche 14 der zweiten Klemmbacke 12.

Erneut Bezug nehmend auf Figur 3 umfasst die hier dargestellte Vorrichtung 10 ein Klemmmaterial 16, welches die Klemmflächen 13, 14 der Klemmbacken 11, 12 ausbildet, die mit der Hautfalte in Kontakt kommen. Das Klemmmaterial 16 an der ersten Klemmbacke 11 und/oder an der zweiten Klemmbacke 12 kann wie dargestellt eine strukturierte Oberfläche aufweisen, beispielsweise mit senkrecht zur Bildebene verlaufenden Rillen und/oder Vertiefungen, um ein Herausrutschen der Hautfalte nach links zu verhindern. Strukturen an den beiden Klemmbacken 11, 12 können wie dargestellt ineinandergreifen. Das Klemmmaterial 16 kann nachgiebig sein und/oder beispielsweise aus Moosgummi ausgestaltet sein, um einerseits einen festen Halt zu ermöglichen und andererseits möglicherweise unangenehme Quetschungen zu verhindern.

Zwischen den Klemmbacken 11, 12 ist ein Federelement 45 angeordnet, welches die Klemmbacken 11, 12 in die geschlossene Position vorspannt. So kann der fixierte Halt der Hautfalte ohne Ausüben einer manuellen Kraft aufrecht erhalten werden.

Im hier dargestellten Beispiel sind die Klemmbacken 11, 12, das Federelement 45 und insbesondere auch die unten beschriebene Schnittstelle 18 einstückig hergestellt, beispielsweise als Spritzgussteil aus Kunststoff.

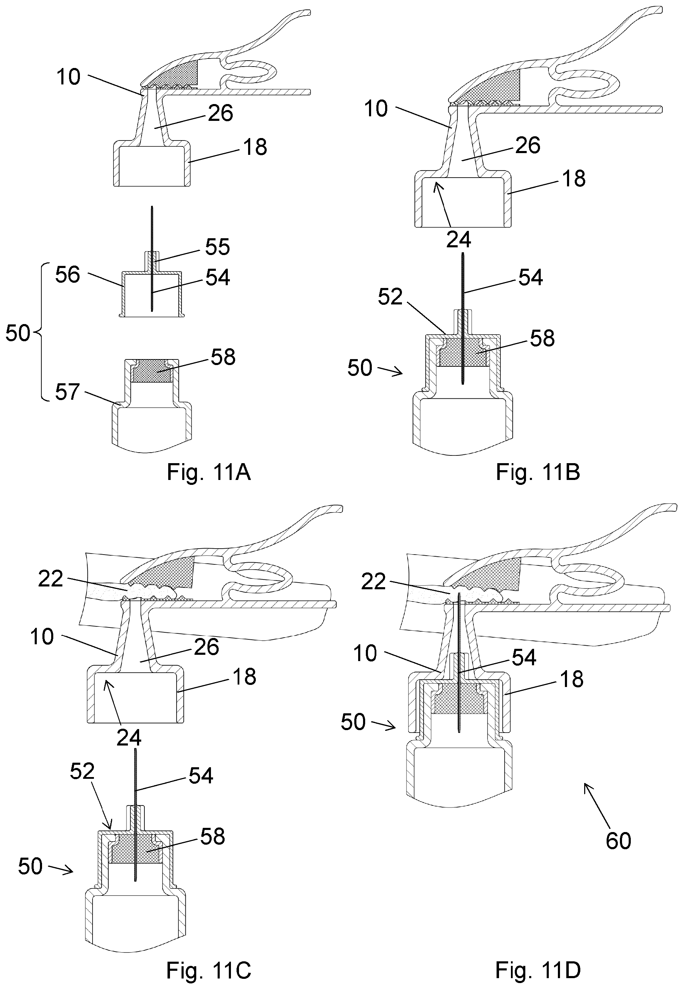

Die Schnittstelle 18 dient in den hier gezeigten Beispielen dem manuell lösbaren Verbinden der Vorrichtung 10 mit dem Injektor 50. Abweichend vom Gezeigten kann auch eine feste Verbindung vorgesehen sein. Die Schnittstelle 18 ist beispielsweise als Steckkappe ausgestaltet, in bzw. auf die der Injektor 50 aufgesteckt werden kann. In Figur 3 ist der aufgesteckte, also mechanisch verbundene Zustand gezeigt. Die Schnittstelle 18 umfasst eine Anschlagfläche 24, auf die in Bezug zu den Figuren 11C und 11D eingegangen wird.

Die Schnittstelle 18 ist mechanisch fest mit der ersten Klemmbacke 11 verbunden und umfasst einen durchgehenden Kanal 26. Die erste Klemmbacke 11 umfasst eine mit dem Kanal verbundene Durchgangsöffnung 27. So kann, wie in Fig. 4 gezeigt, eine Kanüle 54 eines Injektors 50 durch den Kanal 26 und die Durchgangsöffnung 27 hindurch geschoben werden, bis dass die Spitze der Kanüle 54 in die Klemmposition 15 bewegt wird. Figur 6 zeigt, wie die Spitze der Kanüle 54 so auf definierte Weise in die Hautfalte 22 eingestochen werden kann. Alternativ kann eine Vorrichtung zur nadellosen Injektion ganz oder teilweise in den Kanal 26 und ggf. die Durchgangsöffnung 27 hinein bzw. hindurch bewegt werden. Der Kanal 26 verjüngt sich von der Schnittstelle 18 bis hin zur Durchgangsöffnung 27, um ein Einführen z. B. der Kanüle zu erleichtern.

Ein Mittelpunkt der Durchgangsöffnung 27 weist einen Abstand A zu einer Außenkante 28 der ersten Klemmbacke 11 und/oder einer Außenkante 29 der zweiten Klemmbacke 12 auf. Der Abstand A beträgt beispielsweise zwischen 1,5 mm und 3,5 mm.

Der Injektor 50 umfasst eine Kappe 56 mit einem Fortsatz 55 und ein Septum 58. Auf den Injektor 50 wird in Bezug auf die Figuren 11A bis 11D im Detail eingegangen.

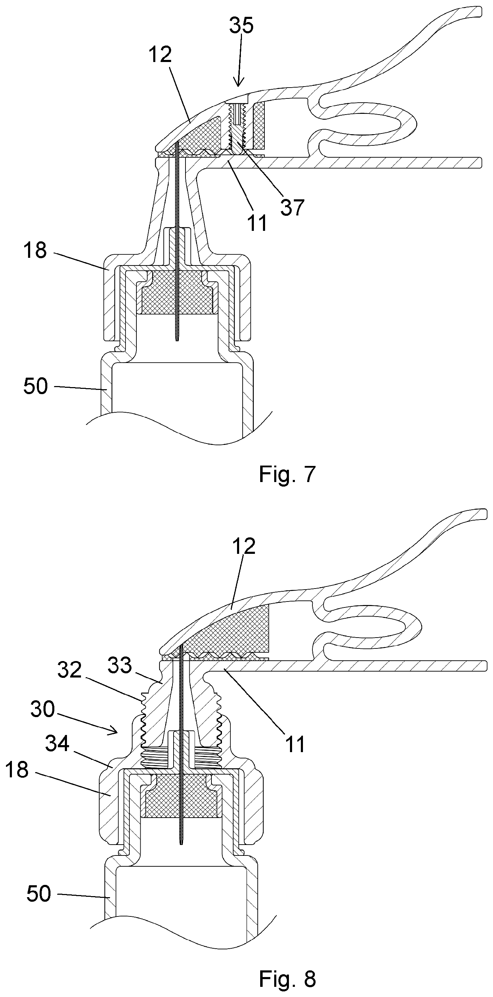

Figur 7 zeigt eine Variante einer Vorrichtung 10, bei der eine zweite Einstelleinrichtung 35 vorhanden ist, um den minimalen Abstand zwischen den Klemmbacken 11, 12 in der Klemmposition in der geschlossenen Position einzustellen. Die zweite Einstelleinrichtung 35 umfasst ein in einer der zwei Klemmbacken 11, 12 angeordnetes, beispielsweise ungefähr senkrecht zur jeweiligen Klemmfläche ausgerichtetes Durchgangsloch mit einem Gewinde und eine in dem Gewinde schraubbare Einstellschraube 37, beispielsweise eine Madenschraube. Die Einstellschraube 37 kann über die Klemmfläche der sie beinhaltenden Klemmbacke 11, 12 hinaus nach vorn gedreht werden und so die jeweils andere Klemmbacke 11, 12 kontaktieren. Auf diese Weise dient es beim Schließen der Klemmbacken 11, 12 als Anschlag. Das Gewinde kann ferner eine Abdeckung aufweisen.

Figur 8 zeigt eine ersten Variante einer Vorrichtung 10 mit einer ersten Einstelleinrichtung 30, um die relative Position des Injektors 50 zur Klemmposition 15 zu beeinflussen. Die erste Einstelleinrichtung 30 umfasst ein Gewinde 32 und ein zugehöriges Schraubelement. Ein erstes Gewindeelement 33 ist mit der ersten Klemmbacke 11 verbunden und ein zweites Gewindeelement 34 wirkt mit dem ersten Gewindeelement 33 zusammen. Die Schnittstelle 18 ist mit dem zweiten Gewindeelement 34 verbunden. Durch relative Drehung entlang einer mittleren Achse der Gewindeelemente 33, 34 wird so ein Abstand zwischen der Schnittstelle 18 und der ersten Klemmbacke 11 verändert.

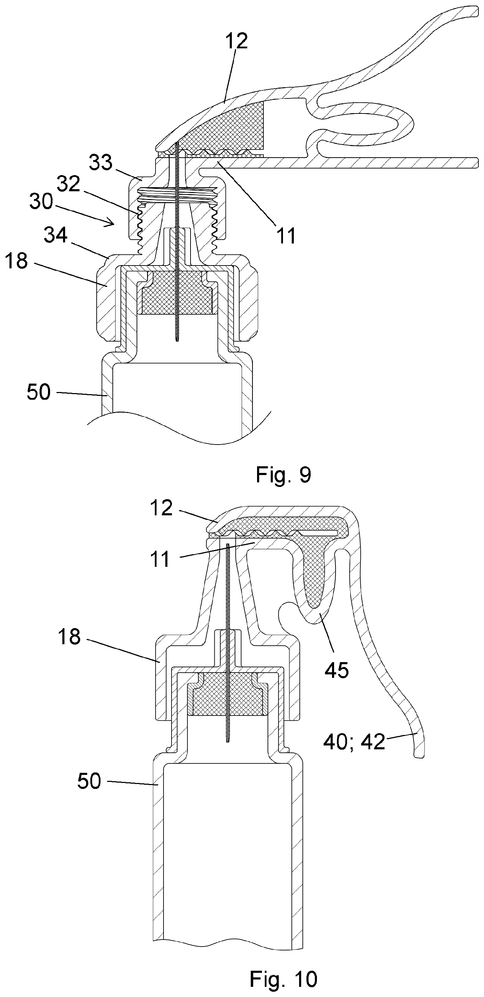

Im hier gezeigten Beispiel ist das erste Gewindeelement 33 mit einem Außengewinde versehen und das zweite Gewindeelement 34 entsprechend mit einem Innengewinde. Der Kanal wird beispielsweise abschnittsweise durch einen Hohlzylinder gebildet. Figur 9 zeigt eine Alternative, in der das erste Gewindeelement 33 ein Innengewinde umfasst und das zweite Gewindeelement 34 entsprechend ein Außengewinde. Der Kanal wird beispielsweise abschnittsweise durch eine Hohlschraube gebildet.

Figur 10 zeigt eine weitere Ausführungsform, in der der Hebel 42 in einem Winkel zwischen 60° und 120°, beispielhaft hier ungefähr 110° zur ersten Klemmbacke 11 ausgerichtet. Dies ermöglicht, mit einer Hand einerseits den Injektor 50 mitsamt der daran befestigten Vorrichtung 10 zu halten und andererseits den Hebel 42 zu betätigen, um eine Hautfalte zu fixieren.

Die Ausführungsformen der Figuren 3, 8 oder 9 sind beliebig mit den Varianten der Figuren 7 und/oder 10 kombinierbar.

Figur 11A bis 11D zeigt einen Ablauf von Verfahrensschritten bei der Verwendung der Vorrichtung 10. In Figur 11A ist ersichtlich, dass der hier beispielhaft dargestellte Injektor 50 einen Injektorkörper 57 und eine Kappe 56 umfasst, die auf den Injektorkörper 56 aufgesteckt werden kann. Die Kappe 56 umfasst die Kanüle 54 und beispielsweise einen Fortsatz 55, in bzw. an dem die Kanüle 54 befestigt ist. Diese Konstruktion erlaubt einen einfachen Wechsel der Kanüle 54, um Verunreinigungen bei der wiederholten Nutzung des Injektors 50 zu verhindern. Die Kanüle ist beidseitig angespitzt. Der Injektorkörper 56 umfasst ein Septum 58, auch als Durchstechmembran bezeichnet, welches beim Aufstecken der Kappe 56 von der unteren Spitze der Kanüle 54 durchstochen wird und auf diese Weise einen abgedichteten Strömungskanal aus dem Injektorkörper 57 bis zur oberen Spitze der Kanüle 54 herzustellen. Das Aufstecken der Kappe 56 ist optional. Figur 11B zeigt den Injektor 50 mit der aufgesteckten Kappe 56.

Figur 1C zeigt die Vorrichtung 10, nachdem die Klemmbacken 11, 12 optional in die geöffnete Position bewegt worden sind (vergl. Figur 4) und eine Hautfalte 22 dazwischen fixiert worden ist. Nun kann der Injektor 50 mit der Schnittstelle 18 verbunden werden, wie in Figur 11D dargestellt ist, damit die Injektion erfolgen kann. In diesem Beispiel kann dabei die Kanüle 54 in die Hautfalte eingestochen werden. Die Vorrichtung 10 und der Injektor 50 bilden somit eine Vorrichtung 60 zur Injektion eines Stoffes.

In den Figuren 11C und 11D ist dargestellt, dass die hier nach oben weisende Vorderfläche der Kappe 56, alternativ eine andere nach vorn weisende Vorderfläche des Injektors 50, ein Formelement 52 ist. Dieses Formelement 52 kommt beim Aufstecken des Injektors 50 in Kontakt mit einer an der Schnittstelle 18 ausgebildeten Anschlagfläche 24.

Dieser Kontakt zwischen dem Formelement 52 und der Anschlagfläche 24 begrenzt die Bewegung des Injektors 50 in die Schnittstelle 18. Auf diese Weise wird die maximale Einstechtiefe der Kanüle in die Hautfalte begrenzt. So kann ein zu tiefes Eindringen sicher verhindert werden. Auf der anderen Seite kann auch ein zu geringes Eindringen verhindert werden, da der Kontakt zwischen dem Formelement 52 und der Anschlagfläche 24 beim Verbinden des Injektors 50 mit der Schnittstelle 18 deutlich spürbar ist, sodass das Verbinden stets unter Herstellung des Kontaktes erfolgt.

### Bezugszeichenliste

| | |
|---|---|
| Vorrichtung | 10 |
| Erste Klemmbacke | 11 |
| Zweite Klemmbacke | 12 |
| Klemmfläche | 13 |
| Klemmfläche | 14 |
| Klemmposition | 15 |
| Klemmmaterial | 16 |
| Schnittstelle | 18 |
| Gelenk | 20 |
| Zeigefinger | 21 |
| Hautfalte | 22 |
| Anschlagfläche | 24 |
| Kanal | 26 |
| Durchgangsöffnung | 27 |
| Außenkante | 28 |
| Außenkante | 29 |
| Erste Einstelleinrichtung | 30 |
| Gewinde | 32 |
| Erstes Gewindeelement | 33 |
| Zweites Gewindeelement | 34 |
| Zweite Einstelleinrichtung | 35 |
| Stellschraube | 37 |
| Betätigungselement | 40 |
| Hebel | 42 |
| Zweiter Hebel | 43 |
| Federelement | 45 |
| Injektor | 50 |
| Formelement | 52 |
| Kanüle | 54 |
| Fortsatz | 55 |
| Kappe | 56 |
| Injektorkörper | 57 |
| Septum | 58 |
| Vorrichtung zur Injektion | 60 |
| Abstand | A |

Die Erfindung kann wie folgt zusammengefasst werden:
Punkt 1: Vorrichtung (10) zur Positionierung eines Injektors (50) in der Nähe eines Gelenks (20), umfassend zwei Klemmbacken (11, 12) zur Fixierung einer Hautfalte (22) in einer Klemmposition (15) und eine Schnittstelle (18) zur mechanischen Verbindung eines Injektors (50).

Punkt 2: Vorrichtung (10) nach dem vorhergehenden Punkt oder einem beliebigen anderen Punkt, dadurch gekennzeichnet, dass die Schnittstelle (18) zur Herstellung einer lösbaren Verbindung des Injektors (50) mit der Vorrichtung (10) eingerichtet ist.

Punkt 3: Vorrichtung (10) nach dem vorhergehenden Punkt oder einem beliebigen anderen Punkt, dadurch gekennzeichnet, dass die Schnittstelle (18) eine Anschlagfläche (24) aufweist, an der ein Formelement des Injektors (50) in einem definierten Abstand zu der Klemmfläche (13, 14) anliegen kann.

Punkt 4: Vorrichtung (10) nach einem der anderen Punkte, dadurch gekennzeichnet, dass die Vorrichtung (10) zwischen der Schnittstelle (18) und einer ersten Klemmbacke (11) der zwei Klemmbacken (11, 12) einen durchgehenden Kanal (26) aufweist.

Punkt 5: Vorrichtung (10) nach dem vorhergehenden Punkt oder einem beliebigen anderen Punkt, dadurch gekennzeichnet, dass der Kanal (26) sich von der Schnittstelle (18) zu der ersten Klemmbacke (11) hin verjüngt.

Punkt 6: Vorrichtung (10) nach Punkt 4 oder Punkt 5 oder einem beliebigen anderen Punkt, dadurch gekennzeichnet, dass ein Abstand (A) zwischen einer Außenkante (28) der ersten Klemmbacke (11) oder einer Außenkante (29) einer zweiten Klemmbacke (12) der zwei Klemmbacken (11, 12) und einem Mittelpunkt einer mit dem Kanal (26) verbundenen Durchgangsöffnung (27) in der ersten Klemmbacke (11) mindestens 1 mm und/oder höchstens 4 mm beträgt.

Punkt 7: Vorrichtung (10) nach einem der anderen Punkte, dadurch gekennzeichnet, dass die Vorrichtung (10) eine erste Einstelleinrichtung (30) aufweist, mit der die relative Position des Injektors (50) in Bezug zu zumindest einer Klemmbacke (11, 12) eingestellt werden kann.

Punkt 8: Vorrichtung (10) nach Punkt 7 oder einem beliebigen anderen Punkt, dadurch gekennzeichnet, dass die erste Einstelleinrichtung (30) ein Gewinde (32) umfasst.

Punkt 9: Vorrichtung (10) nach einem beliebigen anderen Punkt, dadurch gekennzeichnet, dass die Vorrichtung (10) eine zweite Einstelleinrichtung (35) aufweist, mit welcher ein minimaler Abstand zwischen den Klemmbacken (11, 12) eingestellt werden kann.

Punkt 10: Vorrichtung (10) nach einem beliebigen anderen Punkt, dadurch gekennzeichnet, dass die Vorrichtung (10) ein Betätigungselement (40) umfasst, welches manuell betätigt werden kann, um einen Abstand zwischen den Klemmbacken (11, 12) in der Klemmposition (15) zu verringern und/oder zu vergrößern.

Punkt 11: Vorrichtung (10) nach Punkt 10 oder einem beliebigen anderen Punkt, dadurch gekennzeichnet, dass das Betätigungselement (40) als Hebel (42) ausgestaltet ist, wobei ein Drücken des Hebels bewirkt, dass der Abstand vergrößert wird.

Punkt 12: Vorrichtung (10) nach Punkt 11 oder einem beliebigen anderen Punkt, dadurch gekennzeichnet, dass der Hebel (42) in einem Winkel von wenigstens 60° und/oder höchstens 120° zu zumindest einer der Klemmbacken (11, 12) ausgerichtet ist.

Punkt 13: Vorrichtung (10) nach einen der anderen Punkte, dadurch gekennzeichnet, dass die Vorrichtung (10) ein Federelement (45) aufweist, welches die Klemmbacken (11, 12) in eine geschlossene Position vorspannt.

Punkt 14: Vorrichtung (60) zur Injektion eines Stoffes in der Nähe eines Gelenks (20), umfassend die Vorrichtung (10) zur Positionierung eines Injektors (50) gemäß einem der vorhergehenden Punkte und einen mit der Schnittstelle (18) verbundenen oder verbindbaren Injektor (50) zur Injektion eines Stoffes.

Punkt 15: Verfahren zur Verwendung der Vorrichtung (10) zur Positionierung eines Injektors nach einem der Punkte 1 bis 13, umfassend:
- Fixieren einer Hautfalte (22) mit den zwei Klemmbacken (11, 12) der Vorrichtung (10),
- insbesondere Injektion eines Stoffes in die Hautfalte (22) mit einem mit der Schnittstelle (18) der Vorrichtung (10) mechanisch verbundenen Injektor (50).

## Patentansprüche

1. Vorrichtung (10) zur Positionierung eines Injektors (50) in der Nähe eines Gelenks (20), umfassend zwei Klemmbacken (11, 12) zur Fixierung einer Hautfalte (22) in einer Klemmposition (15) und eine Schnittstelle (18) zur mechanischen Verbindung eines Injektors (50),
wobei die Vorrichtung (10) ein Betätigungselement (40) umfasst, welches manuell betätigt werden kann, um einen Abstand zwischen den Klemmbacken (11, 12) in der Klemmposition (15) zu vergrößern, wobei das Betätigungselement (40) als Hebel (42) ausgestaltet ist, wobei ein Drücken des Hebels (42) bewirkt, dass der Abstand vergrößert wird,
**dadurch gekennzeichnet, dass** der Hebel (42) in einem Winkel von wenigstens 60° zu zumindest einer der Klemmbacken (11, 12) ausgerichtet ist, wobei der Hebel (42) in Bezug zu einer Ebene der einen der Klemmbacken (11, 12) in Richtung der Schnittstelle (18) ragt.

2. Vorrichtung (10) nach dem vorhergehenden Anspruch, **dadurch gekennzeichnet, dass** die Schnittstelle (18) zur Herstellung einer lösbaren Verbindung des Injektors (50) mit der Vorrichtung (10) eingerichtet ist.

3. Vorrichtung (10) nach dem vorhergehenden Anspruch, **dadurch gekennzeichnet, dass** die Schnittstelle (18) eine Anschlagfläche (24) aufweist, an der ein Formelement des Injektors (50) in einem definierten Abstand zu der Klemmfläche (13, 14) anliegen kann.

4. Vorrichtung (10) nach einem der vorhergehenden Ansprüche, **dadurch gekennzeichnet, dass** die Vorrichtung (10) zwischen der Schnittstelle (18) und einer ersten Klemmbacke (11) der zwei Klemmbacken (11, 12) einen durchgehenden Kanal (26) aufweist.

5. Vorrichtung (10) nach dem vorhergehenden Anspruch, **dadurch gekennzeichnet, dass** der Kanal (26) sich von der Schnittstelle (18) zu der ersten Klemmbacke (11) hin verjüngt.

6. Vorrichtung (10) nach einem der zwei vorhergehenden Ansprüche, **dadurch gekennzeichnet, dass** ein Abstand (A) zwischen einer Außenkante (28) der ersten Klemmbacke (11) oder einer Außenkante (29) einer zweiten Klemmbacke (12) der zwei Klemmbacken (11, 12) und einem Mittelpunkt einer mit dem Kanal (26) verbundenen Durchgangsöffnung (27) in der ersten Klemmbacke (11) mindestens 1 mm und/oder höchstens 4 mm beträgt.

7. Vorrichtung (10) nach einem der vorhergehenden Ansprüche, **dadurch gekennzeichnet, dass** die Vorrichtung (10) eine erste Einstelleinrichtung (30) aufweist, mit der die relative Position des Injektors (50) in Bezug zu zumindest einer Klemmbacke (11, 12) eingestellt werden kann.

8. Vorrichtung (10) nach dem vorhergehenden Anspruch, **dadurch gekennzeichnet, dass** die erste Einstelleinrichtung (30) ein Gewinde (32) umfasst.

9. Vorrichtung (10) nach einem der vorhergehenden Ansprüche, **dadurch gekennzeichnet, dass** die Vorrichtung (10) eine zweite Einstelleinrichtung (35) aufweist, mit welcher ein minimaler Abstand zwischen den Klemmbacken (11, 12) eingestellt werden kann.

10. Vorrichtung (10) nach einem der vorhergehenden Ansprüche, **dadurch gekennzeichnet, dass** die Vorrichtung (10) ein Federelement (45) aufweist, welches die Klemmbacken (11, 12) in eine geschlossene Position vorspannt.

11. Vorrichtung (60) zur Injektion eines Stoffes in der Nähe eines Gelenks (20), umfassend die Vorrichtung (10) zur Positionierung eines Injektors (50) gemäß einem der vorhergehenden Ansprüche und einen mit der Schnittstelle (18) verbundenen oder verbindbaren Injektor (50) zur Injektion eines Stoffes.

12. Verfahren zur Verwendung der Vorrichtung (10) zur Positionierung eines Injektors nach einem der Ansprüche 1 bis 10, umfassend:
- Fixieren einer Hautfalte (22) mit den zwei Klemmbacken (11, 12) der Vorrichtung (10).
